(19) Europäisches Patentamt
European Patent Office
Office européen des brevets

(11) Publication number: 0 090 063
B1

(12) # EUROPEAN PATENT SPECIFICATION

(45) Date of publication of patent specification: 11.09.85

(51) Int. Cl.⁴: C 07 C 103/44, C 07 C 103/82, C 07 D 295/12 // C07D263/12, C07D263/14

(21) Application number: 82102631.7

(22) Date of filing: 29.03.82

(54) **Process for making N-(2-aminoethyl)amides from oxazolines and amines.**

(43) Date of publication of application:
05.10.83 Bulletin 83/40

(45) Publication of the grant of the patent:
11.09.85 Bulletin 85/37

(84) Designated Contracting States:
BE DE FR GB IT NL

(56) References cited:
US-A-4 105 669
US-A-4 326 067

SYNTHESIS - INTERNATIONAL JOURNAL OF METHODS IN SYNTHETIC ORGANIC CHEMISTRY, No. 7, July 1981, Stuttgart; G.S. POINDEXTER "2-ethyl-4,5-dihydro-1,3-oxazole: a useful aziridine equivalent for the preparation of substituted 1,2-ethanediamines", pp. 541 to 543

ANGEWANDTE CHEMIE, Vol. 78, No. 20, 15 October 1966; W. SEELIGER et al. "Neuere Synthesen und Reaktion cyclischer Imidsäurester", pp. 913 to 927

(73) Proprietor: THE DOW CHEMICAL COMPANY
2030 Dow Center Abbott Road P.O. Box 1967
Midland, MI 48640 (US)

(72) Inventor: Fazio, Michael Joseph
4617 Forestview
Midland Michigan 48640 (US)

(74) Representative: Casalonga, Axel et al
BUREAU D.A. CASALONGA OFFICE JOSSE & PETIT Baaderstrasse 12-14
D-8000 München 5 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 090 063 B1

**Description**

This invention relates to a process for making N-(2-aminoethyl)amides or diamines from a 2-oxazoline or 2-oxazine, or a 2-oxazolinium or oxazinium and an amine.

The formation of N-(2-substituted ethyl)amides from oxazolines and hydrochloric acid is known, as shown by the equation:

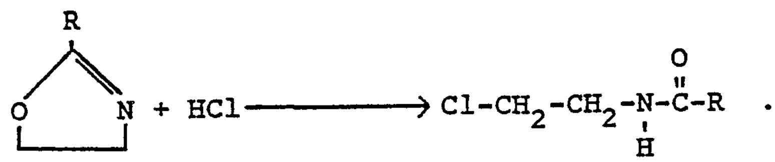

Other substituents such as

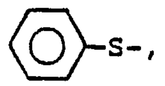

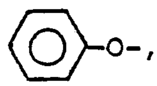

or

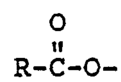

can be substituted for the Cl—.

In *Angew. Chemische* (International Edition) V, No. 10, pp. 875—888 (1966), Seeliger et al. describes the reaction of oxazolines and anilines wherein:

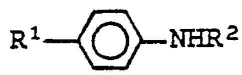

is substituted for HCl in the above reaction.

In *Chemical Abstracts,* 38:12805f, Kormendy et al. disclose the reaction of:

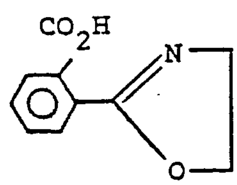

+

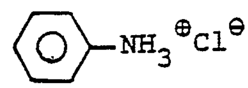

to yield

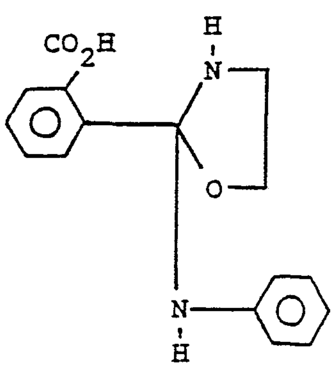

In *Chemical Reviews*, LXXI, No. 5 (1971), pp. 483—505 at page 486, Frump states that when N-(2-bromoethyl)benzamide and diethylamine are boiled together in benzene, 2-phenyl-2-oxazoline is formed.

Rosnati et al. in *Tetrahedron,* IX, pp. 175—82 (1960) reacted ammonia and dimethylamine with a substituted 2-oxazoline to form benzamides.

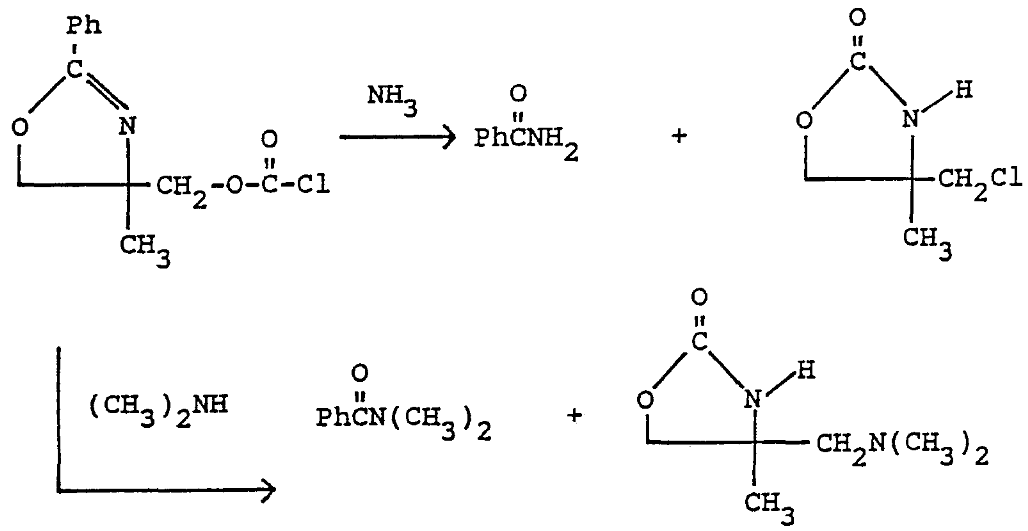


U.S. Patent 4,014,880, Dowd et al., March 29, 1977, discloses the reaction of an oxazoline with ethylene diamine to form 2-ethyl-2-imidazoline and ethanolamine.

U.S. Patent 4,086,274, Kaiser et al., April 25, 1978, discloses a process for preparing N-(2-mercaptoethyl)alkanamide by reaction of a 2-oxazoline and hydrogen sulfide. U.S. Patent 4,086,273, Berazosky et al., April 25, 1978, discloses a process for preparing β-aminoethyl sulfides from aliphatic mercaptans and 2-oxazoline.

The present invention is a process for preparing a N-(2-substituted aminoethyl)amide of the formula:

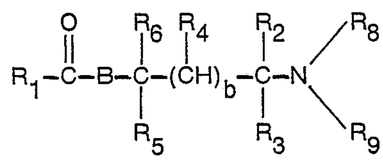

I

characterized by reacting one or more compounds of the formula:

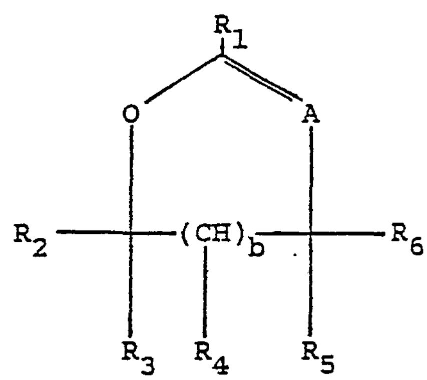

II

with an amine of the formula:

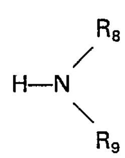

III

wherein A is nitrogen or a quaternary nitrogen of the formula:

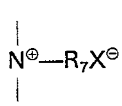

IV

wherein B is

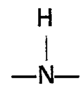

when A is nitrogen and B is

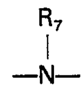

when A is IV; wherein $X^{\ominus}$ is a counterion; and wherein $R_1$—$R_7$ are each hydrogen, an aliphatic or cycloaliphatic or aromatic radical, or an inertly-substituted aliphatic, cycloaliphatic or aromatic radical; and wherein $R_8$ and $R_9$ are each hydrogen, an aliphatic or cycloaliphatic or inertly-substituted aliphatic or cycloaliphatic radical; or $R_8$ and/or $R_9$ are:

$$R_{10}NH_2$$

wherein $R_{10}$ is an aliphatic or cycloaliphatic or an inertly-substituted aliphatic or cycloaliphatic chain containing from 4 to 25 carbon atoms which separates the 2 nitrogen atoms by at least 4 carbon atoms; or $R_8$, $R_9$ and the mediate nitrogen atom form an inertly-substituted or unsubstituted aliphatic heterocyclic ring containing from 4 to 7 members; and wherein b is zero or 1.

In a preferred embodiment, the process is catalyzed by a catalytic amount of a catalyst sufficient to catalyze the reaction; said catalyst being a Lewis acid or a protinic acid with said protonic acid having a non-nucleophilic counterion.

The oxazolines and oxazines used in the process of the invention have the structure:

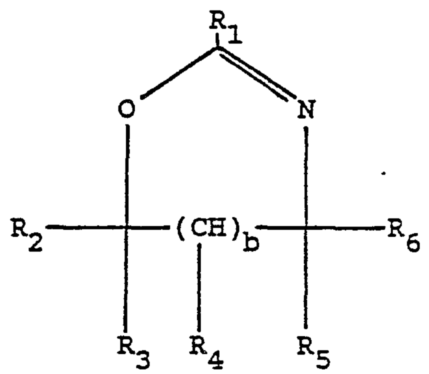

IIa

wherein b is zero for the oxazolines and one for the oxazines. Oxazolines are preferred.

The 2-oxazolines and 2-oxazines of the process of the invention are generally known compounds. Methods for their synthesis are discussed in Frump and Seeliger et al., supra. Specific methods for making the oxazolines are disclosed in U.S. Patent 4,203,900, Kaiser, May 20, 1980.

$R_1$ is hydrogen or an aliphatic or aromatic radical or an inertly-substituted aliphatic or aromatic radical having generally up to 25 carbon atoms. $R_1$ can be a link in a polymer backbone. For example in the structure:

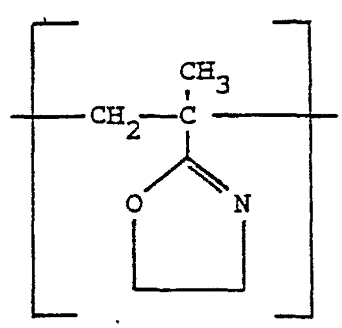

which can be a homopolymer or heteropolymers as described in U.S. Patent 3,505,297, Sheetz et al., April 7, 1970. Comonomers are limited to those with functionality that does not react with the amine, e.g., styrene, vinylpyridine, ethylene, etc.

$R_1$ may also represent a link to a second oxazoline ring to form a bis-oxazoline as exemplified by the structure:

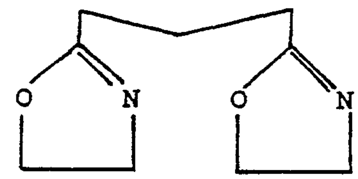

which is disclosed in U.S. Patents 2,569,428, Rowland, September 25, 1951, and 3,419,520, Campbell et al., December 31, 1968.

A key aspect is that $R_1$ is inert when it is exposed to other reactants under the reaction conditions. By inert it is meant that $R_1$ will not react with amines, will not sterically hinder the ring opening reaction, and will not react at a rate faster nor at a rate significantly close to that of the amines with other substituents in its own or other similar molecules. For example, $R_1$ can be hydrogen, methyl, ethyl, undecyl, stearyl, phenyl, benzyl, hydroxyethyl, or p-nitrophenyl. Inert substituents include, for example, the radicals capable of being $R_1$, ethers, thioethers, amides, hydroxy and tertiary amines. $R_1$ is preferably a straight chain aliphatic radical of 1—12 carbon atoms. $R_1$ is most preferably ethyl. $R_2$—$R_3$ and $R_5$—$R_6$ which may be the same or different, have the same definition as $R_1$ except that the most preferred embodiment is hydrogen. $R_4$, of course, does not exist in the oxazoline structure.

Where A is

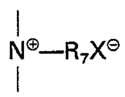

IV

the starting compounds are oxazoliniums (b is zero) or oxaziniums (b is one). $R_1$—$R_6$ have the same definition and preferred embodiments as the oxazolines and oxazines.

The primary difference between the oxazoline-oxazine structures and the oxazolinium/oxazinium structures is that the latter have a quaternized nitrogen with the accompanying radical $R_7$ and the counterion $X^{\ominus}$. Upon reaction, the counterion reacts with a hydrogen made available by the reaction to form HX.

$R_7$ has the same definition as $R_2$ with methyl being most preferred. The oxazoliniums and oxaziniums of this invention are prepared by adding an alkylating agent to the oxazoline or oxazine. This reaction and these salts are discussed in Frump, *Supra* at page 497. Representative alkylating agents are: methyl tosylate, benzyl chloride, and methyl iodide.

The counterion $X^{\ominus}$ is one which will not react with the oxazolines or oxazines under the reaction conditions used.

The amines used in the process of the invention have the structure:

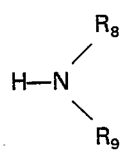

III

$R_8$ and $R_9$, which can be the same or different are hydrogen, an aliphatic or cycloaliphatic or an inertly-substituted aliphatic or cycloaliphatic radical; $R_{10}NH_2$ wherein $R_{10}$ is an aliphatic or cycloaliphatic chain or inertly-substituted aliphatic or cycloaliphatic chain of from 4 to 25 carbon atoms which separate the two nitrogen atoms by at least 4 carbon atoms; or $R_8$, $R_9$ and the mediate nitrogen atom together form an aliphatic or an inertly -substituted aliphatic heterocyclic ring containing four to seven members and preferably five or six members.

Representative examples of $R_8$ and $R_9$1are methyl, ethyl, n-propyl, n-butyl, iso-propyl, 2-methyl butyl, 2-phenyl propyl and cyclohexyl. Methyl and ethyl are preferred. Representative examples of a ring formed by $R_8$, $R_9$ and the mediate nitrogen atom are: N-pyrrolidinyl, 2-methyl-N-pyrrolidinyl, N-piperidinyl, N-piperazinyl and N-morpholinyl.

$R_{10}NH_2$ can be, for example, 4-amino n-butyl, 5-amino, or 4-amino-n-pentyl. The substituents on $R_8$, $R_9$, the rings formerd by $R_8$ and $R_9$ and on $R_{10}$ may be inertly-substituted. By inert it is meant that the substituent group is substantially less nucleophilic than the nitrogen mediate $R_8$ and $R_9$ of III. That is, the substituent reacts with oxazolines not at all or at most at a substantially slower rate than the above cited nitrogen. Examples of inert substituents include: hydroxyl, ethers, aliphatic or aromatic hydrocarbon radicals, esters, tertiary amines, amides and the groups which can be $R_8$ or $R_9$.

$R_8$ and/or $R_9$ can also be a polymeric backbone. Examples include the polyamines such as triethylenetetraamine.

In a preferred process of the invention, the reaction is catalyzed by a catalytic amount of a Lewis acid or

a protonic acid wherein the protonic acid has a non-nucleophilic counterion. By a catalytic amount is meant an amount substantially less than molar equivalency. A catalytic amount is generally less than 5 mole percent based on hte oxazoline. Preferably, the catalyst is present at from about 0.01 mole percent to about 2 mole percent based on the oxazoline.

Lewis acids are well-known to those skilled in the art and are generally defined as a substance that can take up an electron pair to form a covalent bond. Representative examples inlcude $BF_3$, $AlCl_3$, $SnCl_4$, $ZnCl_2$, $FeCl_2$, $H_2WO_3$, $Fe_2SO_4$, $Zn(O_2CCH_3)_2$, $CdCl_2$, $CoCl_2$, and $I_2$. Protonic acids with non-nucleophilic counterions or anions are also a known class of compounds. Protonic acids contain hydrogen. Representative examples include: p-toluenesulfonic acid, sulfuric acid and phosphoric acid. The preferred catalysts are Lewis acids and the most preferred is zinc acetate.

The amino amides formed have the structure:

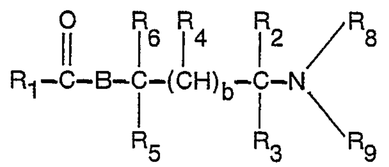 I

wherein $R_1$—$R_{10}$ have the definitions cited above and B is

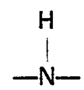

when A is nitrogen and B is

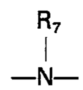

when A is as in formula IV. When oxazolines are reacted, B is

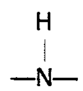

b is zero, and $R_4$ and $R_7$ will not be part of the product.

A preferred reaction is that of 2-ethyl-2-oxazoline with either diethyl- or dimethylamine. The product formed is N-(2-diethylaminoethyl)propionamide or N-(2-dimethylaminoethyl)propionamide, respectively.

The process of the invention may be used to form polymers. For example, the combination of:

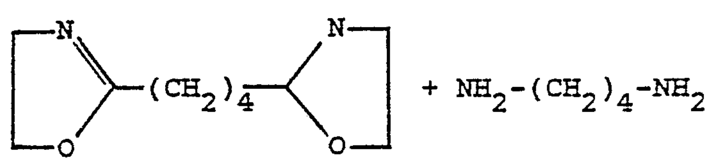

yields alternating monomer units of the structures:

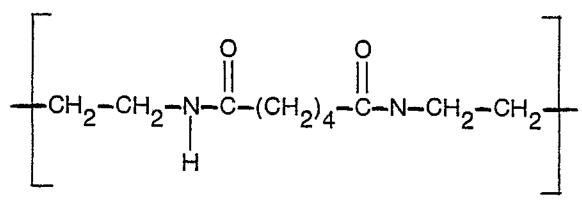

and

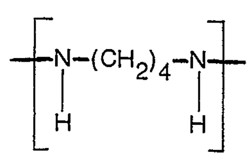

The process of the invention may also be used as a cross-linking reaction. For example, with polymers with pendant oxazolines of the structure:

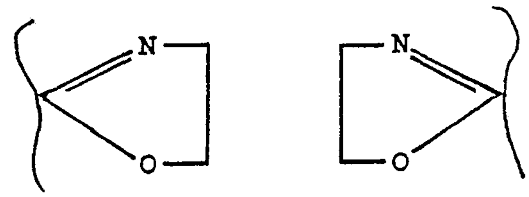

addition of a diamine of the invention such as

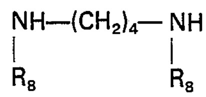

yields:

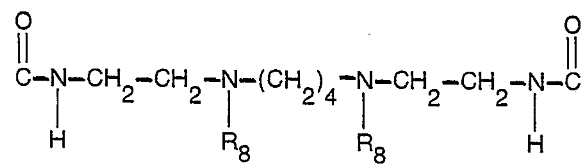

Conversely, bis-oxazolines will cross-link polymers with pendant amine groups.

The process of the invention is carried out at a temperature high enough to permit reaction. The reactants are normally in the liquid state. Preferably, the reaction is carried out at a minimum temperature of about 50°C and more preferably, at a minimum of about 100°C.

The maximum temperature feasible is that at which the reactants thermally decompose or form significant quantities of by-products. Normally the maximum desirable temperature is no more than about 250°C. Preferably, the temperature is at or below about 225°C. Most preferably, the reaction is carried out at a temperature from about 100°C to about 225°C.

The process of the reaction is normally and preferably carried out at autogenous pressure. That is, at atmospheric pressure or, if it is higher, the vapor pressure of the combined reactants at the reaction temperature. However, lower or higher pressures are feasible.

The reaction may be carried out in a solvent inert to the reactants but is preferably done neat. Suitable solvents include toluene, ethers and p-dichlorobenzene.

The reactants are generally added in equimolar amounts, although an excess, for example, of a cheaper reactant can be used. Preferably, the reactants will have an oxazoline/amine ratio of from about 0.9 to about 1.1.

Further reactions of the products of the process of the invention are also of interest. These reactions when combined with the inventive process are also considered inventive and within the scope of the invention. The N-(2-aminoethyl)amides can undergo hydrolysis to diamines and carboxylic acids. For example:

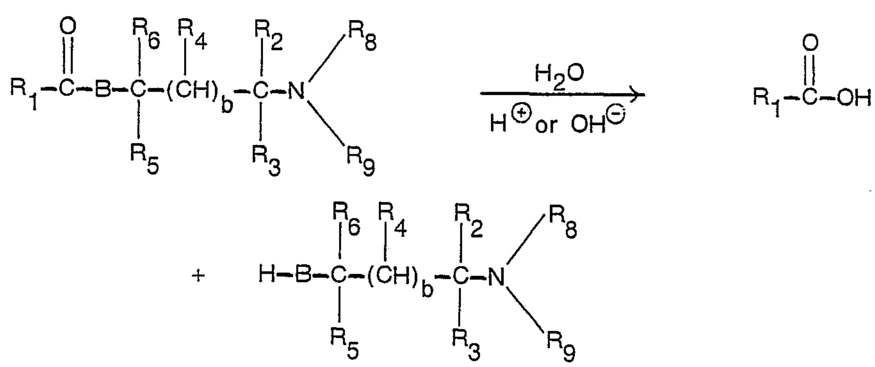


In the case of the reaction of 2-ethyl-2-oxazoline with dimethylamine, the hydrolysis of the amide yields propanoic acid and N,N-dimethylethylenediamine. Fifteen percent aqueous caustic (NaOH or KOH) is the preferred hydrolysis medium.

The amide products of the inventive process also undergo transamidation. That is, the amide is contacted with, for example, a high boiling amine which changes places with the amine functionality of the amide. For example:

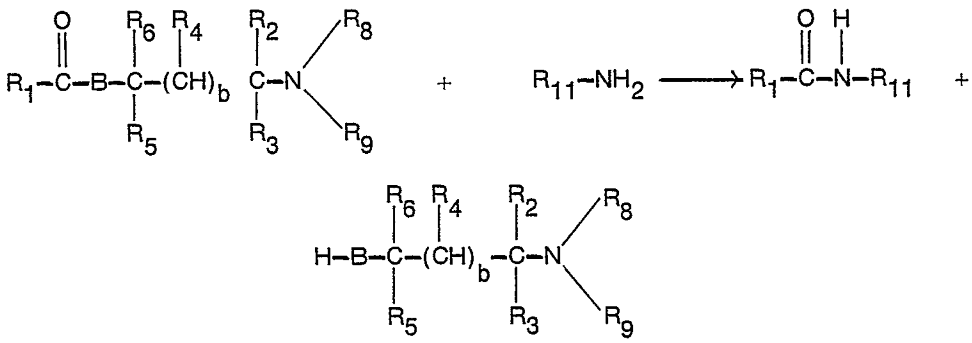

In a preferred embodiment the reaction is:

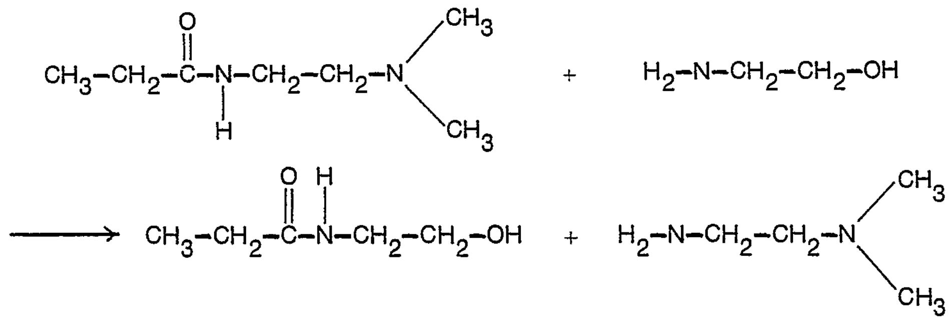

The N-(2-hydroxyethyl)propionamide by-product may then be reacted to form more 2-ethyl-2-oxazoline.

When $R_8$=H and B= $-\overset{H}{\overset{|}{N}}-$,

the amino amide formed may also undergo a cyclization to form an imidazoline. For example:

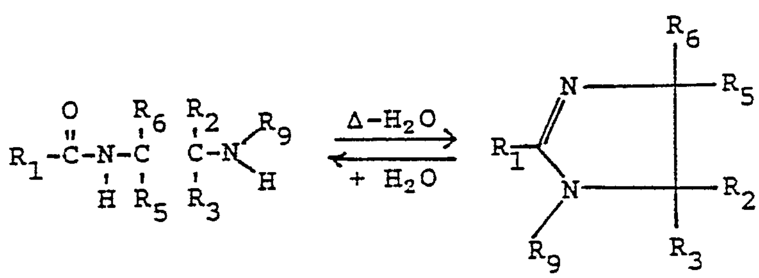


The imidazolines may be hydrolyzed to again form the N-(2-aminoethyl)amide.

The compounds formed by the process of the invention are generally known and have many uses. N-(2-dialkylaminoethyl)alkylamides are taught to retard the ripening of fruit in U.S. Patent 4,148,926. The N,N-dialkylethylenediamines are known to be useful as stabilizers for polystyrene (USP 2,873,264), in leather dyeing (CA 66:76949z), dye acceptors for acrylic and vinyl copolymers (CA 51 15176b), curing agents for polyepoxides (USP 4,201,854), and as intermediates for many other compounds such as procaineamide.

Example 1

A 90 ml stainless steel reactor is charged with 28.18 gm (0.285 mole) of 2-ethyl-2-oxazoline, 20.7 gm (0.284 mole) of diethylamine and 0.54 gm (0.003 mole) of zinc acetate. The reactor is purged with nitrogen, sealed and heated to 225°C. After 19 hours at that temperature and at autogenous pressure, the reactor is cooled to room temperature and the contents of the reactor analyzed. 5.56 Weight percent diethylamine, 1.03 weight percent ethyloxazoline and 81.4 weight percent N-(2-diethylaminoethyl)propionamide are found.

Example 2

Phenyl-2-oxazoline (37 mmoles), diethylamine (37 mmoles) and zinc acetate (0.6 mmole) are charged to a 45 ml reactor and heated to 170°C—175°C for 20 hours at autogenous pressure. Analysis shows 11.9 weight percent unreacted amine, 7.3 weight percent unreacted phenyloxazoline and 57.5 weight percent N-(2-diethylaminoethyl)benzamide.

Example 3

Undecyloxazoline (53 mmoles), diethylamine (104 mmoles) and 0.9 mmole of zinc acetate are reacted under conditions similar to Example 2 to yield 87 percent of theoretical N-(2-diethylaminoethyl)undecylamide.

Examples 4—12

Different catalysts are compared at approximately one mole percent concentrations with reactants and conditions similar to Example 1. Table A gives results as a weight percent of theoretical yield based on the oxazoline.

TABLE A

| Example | Catalyst | % Yield |
|---|---|---|
| 4 | No Catalyst | 7.8 |
| 5 | $H_2WO_3$ | 62.5 |
| 6 | $Fe_2SO_4nH_2O$ | 79.4 |
| 7 | $Zn(CH_3COO)_2{\cdot}2.5H_2O$ | 82.5 |
| 8 | $Zn(CH_3COO)_2$ | 84.4 |
| 9 | $CdCl_2{\cdot}2H_2O$ | 80.2 |
| 10 | $BF_3$·diethylether | 88.5 |
| 11 | $CoCl_2$ | 86.0 |
| 12 | p-toluene sulfonic acid | 86.0 |

Example 13

5.2 Gm (23 mmoles) of dibutylamine and 2.5 gm (25 mmoles) of ethyloxazoline are reacted in a stainless steel tube with 52 mg (0.3 mmole) of zinc acetate at 205°C and autogenous pressure for 24 hours. Yield determined by analysis of the crude product is 85 percent N-(2-dibutylaminoethyl)propionamide.

Example 14

Under the same conditions as Example 13, 2.5 gm (25 mmoles) of 2-ethyl-2-oxazoline, 1.84 gm (25 mmoles) of t-butylamine and 49.4 mg (0.27 mmole) of zinc acetate are reacted to yield 64 percent N-(2-t-butylaminoethyl)propionamide.

Example 15

Under the same conditions as Example 13, 2.5 gm (25 mmoles) of 2-ethyl-2-oxazoline, 2.2 gm (25 mmoles) of morpholine and 0.59 mg of zinc acetate are reacted to yield 83 percent N-(2-morpholinylethyl)propionamide.

Example 16

28.9 Gm of 2-ethyl-2-oxazoline, 17.4 gm of monoethanolamine and 0.71 gm of zinc acetate are charged to a 90 ml stainless steel reactor. The mixture is heated at autogenous pressure at 200°C for 20 hours. Analysis shows three major components: N-(N-2-hydroxyethyl-2-aminoethyl)propionamide; 1-(2-hydroxyethyl)-2-ethylimidazoline and N-aminoethyl-N-hydroxyethyl propionamide. Hydrolysis of this mixture yields a mixture of N-(2-hydroxyethyl)ethylenediamine, propionic acid and a small amount of unreacted monoethanolamine.

**Claims**

1. A process for preparing a N-(2-substituted aminoethyl)amide of the formula:

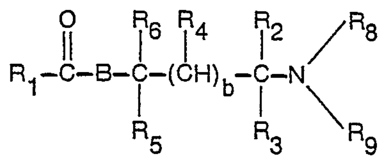

I

characterized by reacting one or more compounds of the formula:

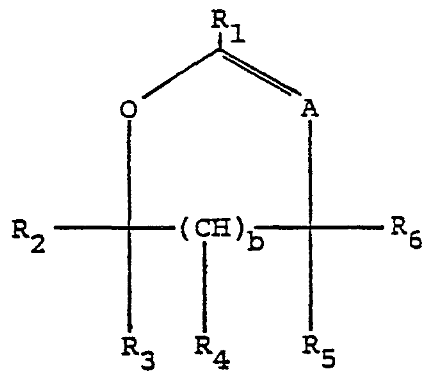

II

with an amine of the formula:

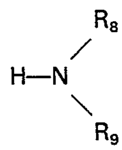

III

and wherein A is nitrogen or a quaternary nitrogen of the formula:

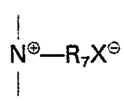

IV

wherein B is

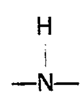

when A is nitrogen and B is

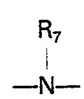

when A is IV; wherein $X^{\ominus}$ is a counterion; and wherein $R_1$—$R_7$ are each hydrogen, an aliphatic or cycloaliphatic or aromatic radical, or an inertly-substituted aliphatic, cycloaliphatic or aromatic radical; and wherein $R_8$ and $R_9$ are each hydrogen, an aliphatic or cycloaliphatic or inertly-substituted aliphatic or cycloaliphatic radical; or $R_8$ and/or $R_9$ are:

$$R_{10}NH_2$$

wherein $R_{10}$ is an aliphatic or cycloaliphatic or an inertly-substituted aliphatic or cycloaliphatic chain containing from 4 to 25 carbon atoms which separates the 2 nitrogen atoms by at least 4 carbon atoms; or $R_8$, $R_9$ and the mediate nitrogen atom form an inertly-substituted or unsubstituted aliphatic heterocyclic ring containing from 4 to 7 members; and wherein b is zero or 1.

2. The process of Claim 1 and further characterized in that the process is catalyzed by a catalytic amount of a Lewis acid or a protonic acid, said prtonic acid having a non-nucleophilic counterion.

3. The process of Claim 1 and further characterized in that the reaction is carried out at a temperature of from 50°C to 250°C.

4. The process of Claim 1 and further characterized by:

(a) hydrolyzing I to form an amine and a carboxylic acid; and

(b) recovering said amine.

5. The process of Claim 1 and further characterized in that the $R_9$ is hydrogen and including the additional steps of:

(a) heating to dehydrate I and form an imidazoline of the structure

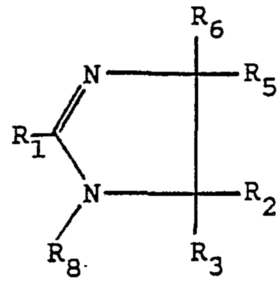

;

and

(b) recovering said imidazoline.

**Patentansprüche**

1. Ein Prozeß zur Darstellung eines N-(2-substituiertes Aminoäthyl)amid mit der Formel:

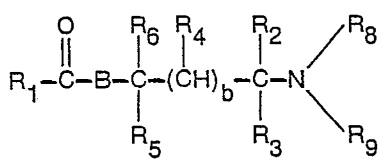

I

charakterisiert durch die Reaktion einer oder mehrerer Verbindungen mit der Formel:

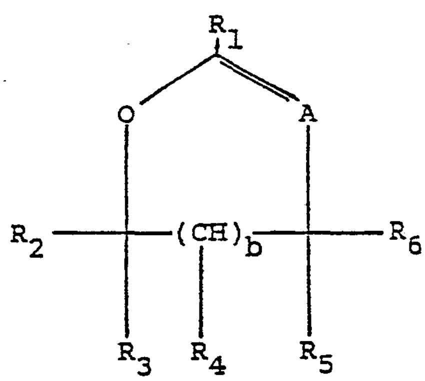

II

mit einem Amin mit der formel:

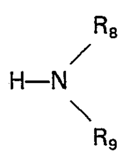

III

und wobei A ein Stickstoff oder ein Quaternärer Stickstoff ist mit der Formel:

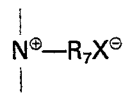

IV

wobei B ist

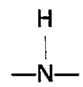

wenn A ein Stickstoffatom ist und B ist

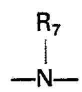

wenn A IV ist; wobei $X^{\ominus}$ ein Gegenion ist; und wobei von $R_1$—$R_7$ jeder Wasserstoff ein alyphatisches oder cyckloalyphatisches oder aromatisches Radikal, oder ein inertsubstituiertes alyphatisches, cyckloalyphatisches oder aromatisches Radikal ist; und wobei $R_8$ und $R_9$ jedes ein Wasserstoff, ein alyphatisches oder cyckloalyphatisches oder inertsubstituiertes alyphatisches oder cyckloalyphatisches Radikal ist; oder $R_8$ und/oder $R_9$ sind:

$$R_{10}NH_2$$

wobei $R_{10}$ ein alyphatisches oder cyckloalyphatisches oder eine inertsubstituierte alyphatische oder cyckloalyphatische Kette ist enthaltend von 4 bis 25 Kohlstoffatome, welche die zwei Stickstoffatome mit mindestens 4 Kohlenstoffatomen trennen; oder $R_8$, $R_9$ und das mittlere Stickstoffatom einen inertsubstituierten oder unsubstituierten alyphatischen heterocyclischen Ring bilden, welcher von 4 bis 7 Glieder enthält; und wobei b eine Null oder 1 ist.

2. Der Prozeß vom Anspruch 1 und weiter charakterisiert in dem der Prozeß durch eine katalytische Menge einer Lewis Säure oder Protonsäure katalysiert wird und die genannte Protonsäure ein nichtnucleophiles Gegenion hat.

3. Der Prozeß vom Anspruch 1 und weiter charakterisiert in dem die Reaktion bei Temperaturen von 50°C bis 250°C durchgeführt wird.

4. Der Prozeß vom Anspruch 1 und weiter charakterisiert durch:

(a) das Hydrolysieren von I um ein Amin und Carboxylsäure zu bilden; und

(b) Isolierung des genannten Amins.

5. Der Prozeß vom Anspruch 1 und weiter charakterisiert in dem $R_9$ ein Wasserstoff ist und die folgenden Schritte einschließt:

(a) Erhitzen von I zum Dehydrieren und Bildung eines Imidazolin mit der Struktur

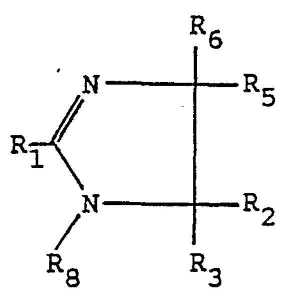

; und

(b) Isolierung des genannten Imidazolin.

**Revendications**

1. Procédé pour préparer un N-(amino 2-éthyl substitué) amide ayant la formule:

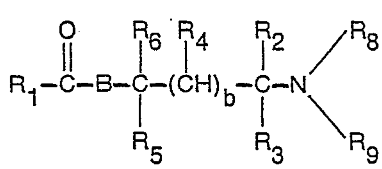

I

caractérisé par la réaction d'un ou plusieurs composés ayant la formule:

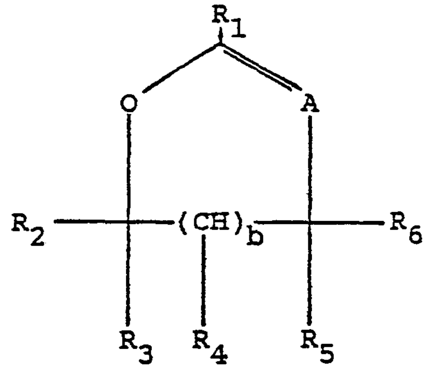

II

avec une amine ayant la formule:

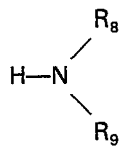

III

et dans laquelle A est un atome d'azote ou un atome d'azote quaternaire de formule:

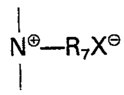

IV

dans laquelle B est

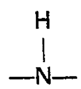

quand A est un atome d'azote, et B est

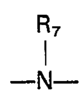

quand A est IV; où $X^{\ominus}$ est un ion opposé; et dans laquelle $R_1$ à $R_7$ sont chacun un atome d'hydrogène, un radical aliphatique ou cyclo-aliphatique ou aromatique, ou bien un radical aliphatique, cyclo-aliphatique ou aromatique substitués par un reste inerte; et dans laquelle $R_8$ et $R_9$ sont chacun un atome d'hydrogène, un radical aliphatique ou cyclo-aliphatique ou bien un radical aliphatique ou cycloaliphatique substitués par un reste inerte; ou bien $R_8$ et/ou $R_9$ sont:

$$R_{10}NH_2$$

dans laquelle $R_{10}$ est une chaîne aliphatique ou cyclo-aliphatique ayant de 4 à 25 atomes de carbone ou bien une chaîne aliphatique ou cyclo-aliphatique de 4 à 25 atomes de carbone substitué par des radicaux inertes, ladite chaîne séparant les 2 atomes d'azote par un moins 4 atomes de carbone; ou bien $R_8$ et $R_9$ et l'atome d'azote intermédiaire forment un noyau hétérocyclique aliphatique, contenant de 4 à 7 éléments, substitué par des radicaux inertes ou non substitué; et dans laquelle b est 0 ou 1.

2. Procédé selon la revendication 1, caractérisé en plus par le fait que le procédé est catalysé par une quantité catalytique d'un acide de Lewis ou d'un acide protonique, ledit acide protonique ayant un ion opposé non-nucléophile.

3. Procédé selon la revendication 1, caractérisé par le fait que la réaction est effectuée à une température allant de 50°C à 250°C.

4. Procédé selon la revendication 1, caractérisé en outre par:

(a) l'hydrolyse de I pour former une amine et un acide carboxylique: et

(b) la récupération de ladite amine.

5. Procédé selon la revendication 1, caractérisé en outre par le fait que $R_9$ est un atome d'hydrogène et qu'il comprend les stades supplémentaires:

(a) de chauffage pour déshydrater I et former une imidazoline ayant la structure:

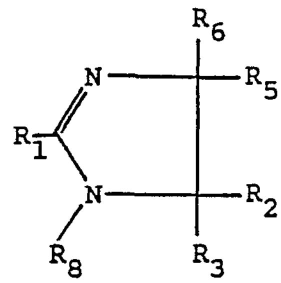

; et

(b) la récupération de ladite imidazoline.